Europäisches Patentamt

**European Patent Office**

Office européen des brevets

(11) Numéro de publication: **0 291 363**
A2

# (12) DEMANDE DE BREVET EUROPEEN

(21) Numéro de dépôt: 88400234.6

(22) Date de dépôt: 02.02.88

(51) Int. Cl.⁴: **G 01 N 33/28**

(30) Priorité: 10.02.87 FR 8701592

(43) Date de publication de la demande:
**17.11.88 Bulletin 88/46**

(84) Etats contractants désignés: **DE ES GB IT**

(71) Demandeur: **REGIE NATIONALE DES USINES RENAULT
Boîte postale 103 8-10 avenue Emile Zola
F-92109 Boulogne-Billancourt (FR)**

(72) Inventeur: **Warenghem, Michel
8, rue de l'Abbé Saint Pierre
F-92150 Suresnes (FR)**

**Fauconnet, Jean-François
84, rue Voltaire
F-92500 Rueil-Malmaison (FR)**

**Bellard, Jean-Pierre
12, Allée de la Sablière
F-78170 La Celle Saint Cloud (FR)**

**Labaune, Camille
8, rue du 4 Septembre
F-78112 Fourqueux (FR)**

(54) **Dispositif de mesure de la concentration de matières charbonneuses dans une huile pour moteur diesel.**

(57) L'invention concerne un dispositif de mesure de la concentration de matières charbonneuses dans une huile pour moteur Diesel, comportant un boîtier renfermant un élément sensible capacitif destiné à être immergé dans l'huile, et dont la capacité varie en fonction de la constante diélectrique de l'huile, caractérisé en ce que cet élément sensible (3) est constitué par deux électrodes parallèles, l'une d'elles connectée à la masse et dite de blindage (4) est réalisée par deux plaques métalliques parallèles reliées entre elles et l'autre dite active (5) placée entre ces deux plaques et isolée électriquement sur presque toute sa surface est reliée à un circuit électronique de traitement (7) destiné à mesurer la concentration de matières charbonneuses dans l'huile à partir de la capacité existant entre lesdites électrodes.
**Applications aux véhicules automobiles.**

FIG.10

# Description

## DISPOSITIF DE MESURE DE LA CONCENTRATION DE MATIERES CHARBONNEUSES DANS UNE HUILE POUR MOTEUR DIESEL

L'invention concerne un dispositif de mesure de la concentration de matières charbonneuses dans une huile pour moteur Diesel.

Le principe de mesure repose sur la variation de la constante diélectrique de l'huile moteur en fonction du pourcentage de matières charbonneuses qu'elle renferme. Ces matières charbonneuses provenant du moteur altèrent les propriétés de lubrification de l'huile dont il est nécessaire de faire la vidange après un certain nombre de kilomètres parcourus par le véhicule. Ce nombre de kilomètres varie en fonction des caractéristiques du moteur et de ses conditions de fonctionnement - circulation urbaine ou rurale, par exemple. C'est pourquoi il est intéressant de connaître le degré d'usure de l'huile pour faire la vidange à bon escient et non au bout d'un kilométrage fixe a priori, correspondant rarement à une réelle détérioration de l'huile.

Un exemple de dispositif de détection de la détérioration de l'huile d'un moteur est décrit dans la demande de brevet européen N° 0 080 632. Il comprend un capteur capacitif réalisé à partir de deux électrodes ajustables. L'inconvénient de ce dispositif est que la capacité du capteur est relativement élevée.

L'invention propose un dispositif dont l'élément sensible présente une faible capacité et une fréquence de fonctionnement élevée pour diminuer l'impédance interne et donc rendre le dispositif indépendant de la température ambiante.

L'objet de l'invention est un dispositif de mesure de la concentration de matières charbonneuses dans une huile pour moteur Diesel comportant un boîtier renfermant un élément sensible capacitif, dont la capacité varie en fonction de la constante diélectrique de l'huile, destiné à être immergé dans l'huile, caractérisé en ce que cet élément sensible est constitué par deux électrodes parallèles, l'une d'elles connectée à la masse et dite de blindage est réalisée par deux plaques métalliques parallèles reliées entre elles et l'autre dite active placée entre ces deux plaques et isolée électriquement sur presque toute sa surface est reliée à un circuit électronique de traitement destiné à mesurer la concentration de matières charbonneuses dans l'huile à partir de la capacité existant entre lesdites électrodes.

Selon une autre caractéristique de l'invention, le dispositif est monté en dérivation du circuit d'huile, par une entretoise avec piquage, placé entre le carter et le filtre à huile.

Selon une autre caractéristique de l'invention, le dispositif est monté en série avec le circuit d'huile, directement entre le carter et le filtre à huile.

D'autres caractéristiques et avantages de l'invention apparaîtront à la lecture de la description qui suit, illustrée par les figures suivantes représentant :
    - les figures 1 et 2 : un premier mode de réalisation du dispositif selon l'invention, monté en dérivation du circuit d'huile ;
    - les figures 3 et 4 : un second mode de réalisation du dispositif selon l'invention, monté en série avec le circuit d'huile ;
    - les figures 5 à 9 : une seconde version du second mode de réalisation ;
    - les figures 10 à 15 : un troisième mode de réalisation du dispositif selon l'invention.

Les éléments identiques assurant les mêmes fonctions en vue des mêmes résultats portent les mêmes références dans les différentes figures.

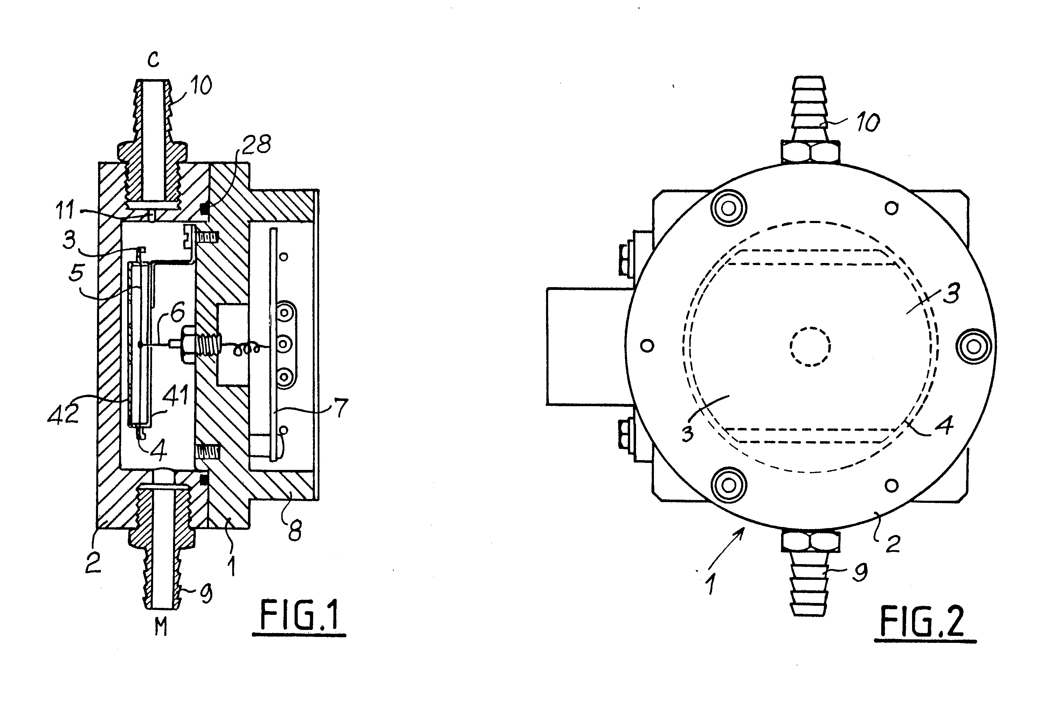

Les figures 1 et 2 représentent un premier mode de réalisation du dispositif selon l'invention, monté en dérivation du circuit d'huile par piquage sur le carter cylindre avec retour sur le carter inférieur, selon une vue en coupe et une vue de côté respectivement.

Le dispositif selon l'invention comporte un boîtier 1 dont une partie 2, cylindrique sur la figure 1, renferme un élément sensible capacitif 3, dont la capacité varie en fonction de la constante diélectrique de l'huile dans laquelle il est immergé. Cet élément sensible 3 est constitué par une première électrode 4 dite de blindage et par une seconde électrode 5 dite active, parallèle à la première. Cette première électrode 4 est réalisée par deux plaques parallèles 41 et 42 métalliques reliées entre elles sur leurs pourtours et reliées au boîtier 1 du dispositif lui-même connecté à la masse. Leur distance de séparation est déterminée pour éviter l'encrassement de l'élément sensible. L'électrode active 5 est placée entre les deux plaques 41 et 42 et est isolée électriquement sur presque toute sa surface pour éviter les court-circuits avec les plaques 41 et 42 dus par exemple aux particules métalliques présentes dans l'huile ou aux gouttes d'eau. Cette électrode active 5 peut être en cuivre recouverte d'une couche de matériau isolant, tel que du Kapton, sur ses deux faces, sauf en un point servant de connexion électrique avec un circuit électronique 7 de traitement, destiné à mesurer la concentration de matières charbonneuses dans l'huile, à partir de la capacité existant entre les électrodes 4 et 5. Ce circuit 7 est logé dans une autre partie 8 du boîtier 1, rendue étanche par un point 28 par exemple.

Un premier flexible 9 assure le passage de l'huile prélevée dans le moteur par piquage et un second flexible 10 assure son retour vers le carter inférieur. Au niveau du branchement de ce flexible 10 sur le boîtier 1, la partie 2 de celui-ci comporte un gicleur 11 pour maintenir sous pression le débit d'huile de façon à éviter l'encrassement du dispositif et de favoriser l'évacuation rapide des bulles d'air. De façon préférentielle, l'élément sensible 3 est situé un peu en dehors de l'écoulement de l'huile entre les deux flexibles 9 et 10, tout en étant totalement immergé dans l'huile afin d'éviter les problèmes de bulles d'air entre électrodes. On a ainsi une information continue dès que le moteur tourne.

En raison de cette disposition en dérivation, le dispositif peut être placé sous le capot moteur loin des températures élevées dues au fonctionnement du moteur. Lorsque le véhicule roule, le dispositif se trouve refroidi par la vitesse. Après chaque arrêt du moteur, le capteur se vide. Tout ce qui vient d'être décrit sur sa constitution évite l'encrassement, facilite le dégazage, permet de s'affranchir de l'émulsion et d'être indépendant du débit et de la pression d'huile.

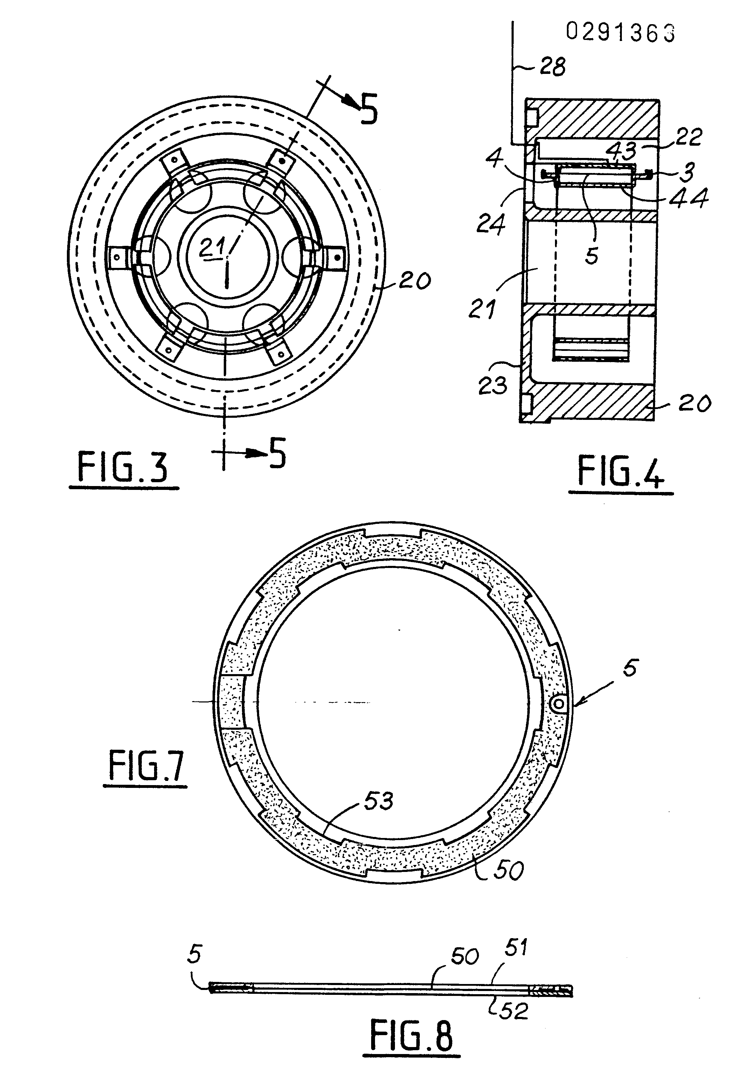

Les figures 3 et 4 représentent, respectivement selon une vue de côté et une vue en coupe, un second mode de réalisation du dispositif selon l'invention, monté en série avec le circuit d'huile. Il comporte un boîtier cylindrique 20 disposé directement entre le carter et le filtre à huile, par entretoise sans flexible. Le boîtier 20 comporte une première cavité cylindrique centrale 21 et une deuxième cavité en couronne 22 autour de la première cavité, celle-ci assurant le passage de l'huile du carter vers le filtre et celle-là assurant le retour de l'huile filtrée vers le carter ; de plus, la face 23 du boîtier 20 en contact avec le carter est percée d'ouvertures 24 au niveau de la couronne 22 pour laisser passer l'huile du moteur vers le filtre.

L'élément sensible capacitif 3 immergé dans l'huile est réalisé à partir d'électrodes 4 et 5 parallèles en forme de cylindres concentriques placés dans la cavité en couronne 22. L'électrode 4 dite de blindage est réalisée par deux plaques cylindriques 43 et 44 concentriques, connectées entre elles à leurs extrémités et reliées au boîtier qui est à la masse.

L'électrode 5 dite active est réalisée par un ruban concentrique avec les plaques 43 et 44, en même matériau que dans le premier mode de réalisation représenté figures 1 et 2, et relié par un câble blindé 28 à un circuit électronique de traitement placé dans un endroit, sous le capot du véhicule, où la température ambiante n'est pas trop élevée.

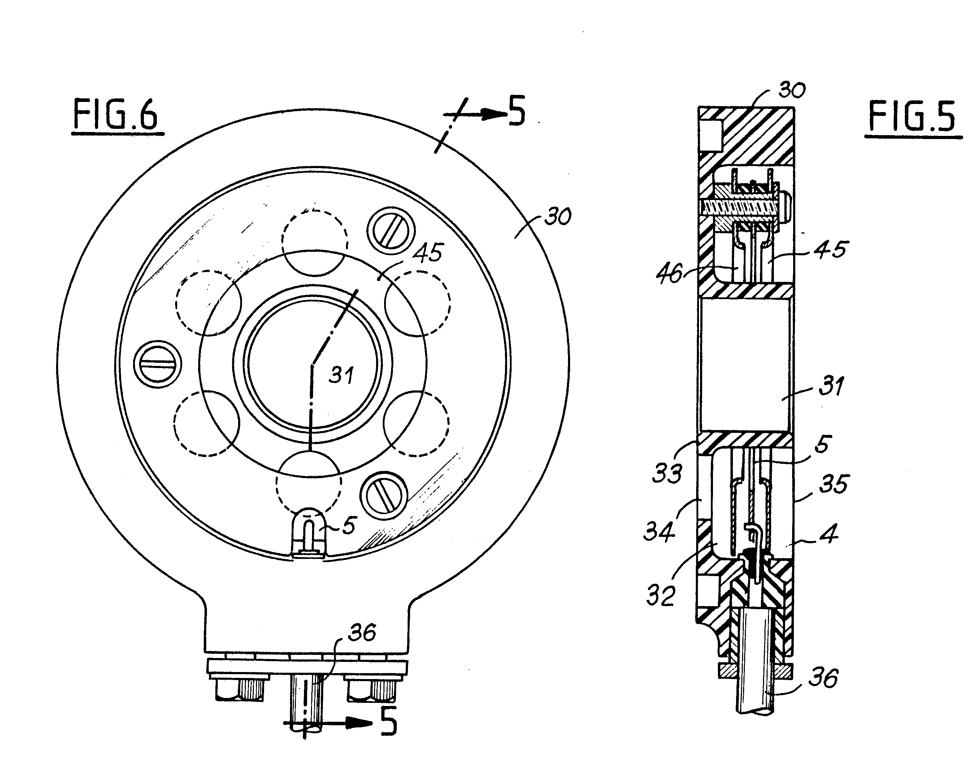

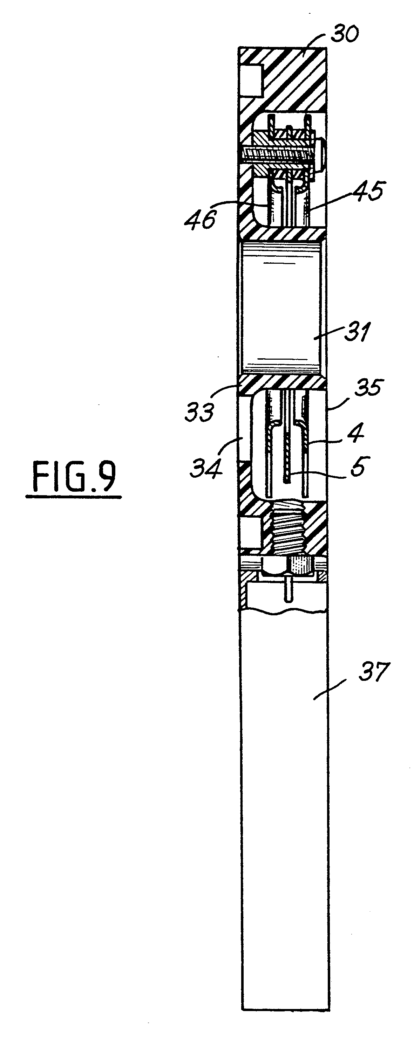

Une seconde version de ce second mode de réalisation d'un dispositif monté en série avec le circuit d'huile est représentée par les figures 5, 6, 7, 8 et 9. Selon cette version, le dispositif comporte un boîtier 30 cylindrique disposé entre le carter et le filtre à huile par entretoise sans flexible. Le boîtier comporte une cavité cylindrique centrale 31 s'adaptant à la sortie du filtre à huile et une cavité 32 en forme de couronne réalisée autour de la première cavité. La face 33 du boîtier en regard du carter est percée d'ouvertures 34 pour laisser passer l'huile qui vient envahir la cavité 32 où est disposé l'élément sensible capacitif 3. l'huile circule ensuite vers le filtre à huile en passant par les ouvertures 35 situées de l'autre côté du boîtier 30. Après passage dans le filtre, l'huile alors filtrée retourne dans le carter par la cavité centrale 31. Cette version diffère essentiellement de la première par la disposition de l'élément sensible capacitif 3 qui est perpendiculaire au débit d'huile dont les électrodes de blindage 4 et active 5 sont en forme de couronnes parallèles et concentriques. L'électrode active 5 est réalisée à partir d'une feuille 50 de cuivre, isolée électriquement sur ses deux faces par deux feuilles 51 et 52 de matériau diélectrique - du Kapton par exemple - comme le montrent les figures 7 et 8. L'électrode de blindage 4 est constituée par deux plaques métalliques 45 et 46, parallèles, en forme de couronne et concentriques, reliées entre elles sur leurs deux circonférences. La figure 7 montre que la couronne de cuivre 50 de l'électrode active 5 comporte des crans 53 au niveau des points de connexion des deux plaques métalliques de l'électrode de blindage 4 pour éviter les court-circuits. Cette électrode active 5 est connectée électriquement à un circuit électronique de traitement par l'intermédiaire d'un câble blindé 36 pour des raisons d'isolement thermique. Ce circuit de traitement peut ainsi être logé sous le capot dans un endroit à l'abri des élévations de température dues au fonctionnement du moteur.

Sur la figure 9 est représenté un dispositif selon l'invention, pour lequel le circuit électronique 37 de traitement est accolé au boîtier 30 contenant l'élément sensible capacitif 3, qui est alors relié directement, sans l'intermédiaire d'un câble blindé, au circuit 37.

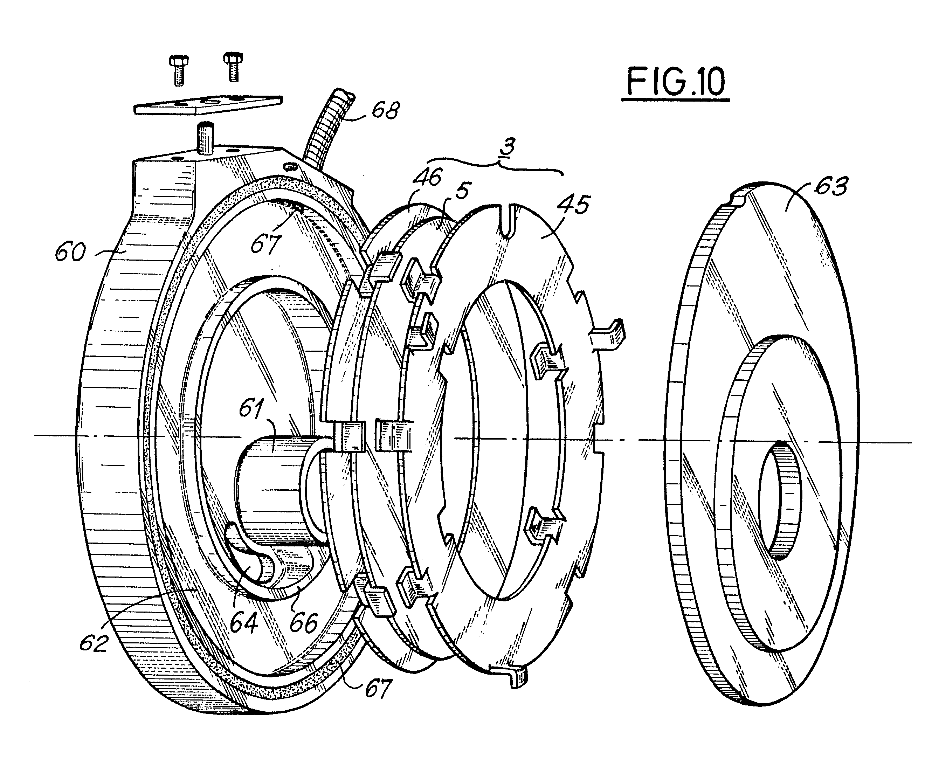

Un troisième mode de réalisation du dispositif selon l'invention est représenté sur les figures 10 à 15.

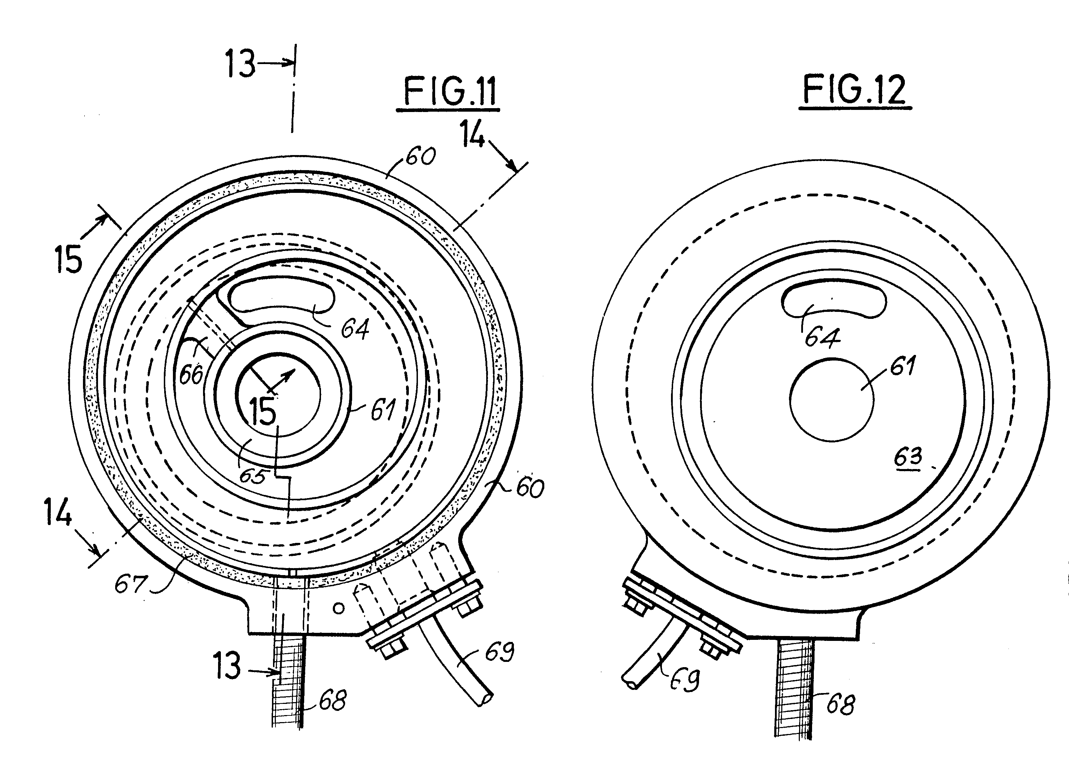

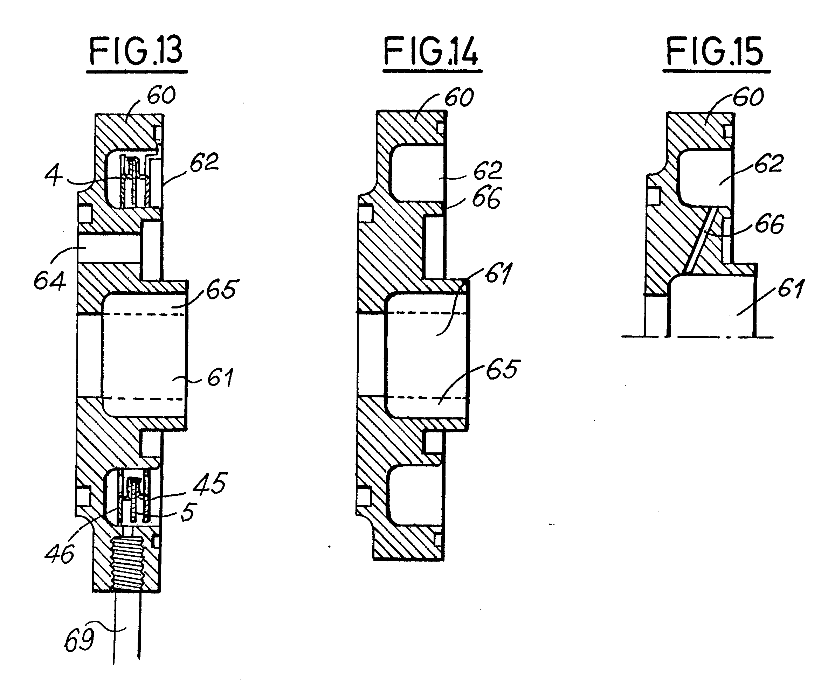

La figure 10 représente une vue éclatée de la partie sensible du dispositif, hormis le circuit électronique de traitement. La figure 11 représente une vue de cette partie, du côté du moteur et la figure 12 une vue du côté du filtre à huile. La figure 13 représente une vue de la figure 11 selon une coupe 13-13 ; la figure 14 représente une vue de la figure 11 selon une coupe 14-14 et la figure 15 une coupe partielle de la figure 11.

Le dispositif comporte un boîtier 60, placé entre le carter et le filtre à huile, comprenant une première cavité cylindrique centrale 61 de diamètre légèrement supérieur au diamètre du tube de sortie d'huile du filtre à huile, et une seconde cavité 62 en couronne dans laquelle est logée l'élément sensible capacitif 3 réalisé comme dans la seconde version du deuxième mode de réalisation décrit.

Il comprend une électrode active 5 en forme de couronne placée entre deux plaques métalliques parallèles 45 et 46 constituant l'électrode de blindage 4.

Dans l'exemple de la figure 10, le boîtier est en deux parties, avec un couvercle 63. L'huile venant du carter pénètre dans le boîtier 60 par une ouverture 64. En raison du montage du boîtier, l'huile passe ensuite dans une chambre de transuilisation 65 existant entre la paroi de la cavité centrale 61 et la paroi du tube de sortie de l'huile hors du filtre à huile, représentée en traits pointillés sur les figures 13 à 15. Après séjour dans cette chambre de tranquilisation 65, l'huile est peu agitée lorsqu'elle pénètre dans la cavité 62 pour immerger l'élément capacitif 3, par un fin canal 66 creusé entre les deux cavités 61 et 62. Enfin, l'huile sort du dispositif selon l'invention par un gicleur 67 prévu dans la paroi extérieure du boîtier, dans un flexible 68 qui la conduit au carter inférieur du moteur.

Sur la figure 13, on voit que l'électrode de blindage 4 est reliée au boîtier 60 qui est à la masse du véhicule et l'électrode active 5 est reliée par un câble blindé 69 à un circuit électronique de traitement que l'on place judicieusement loin des températures élevées du moteur en fonctionnement.

Dans ces différents modes de réalisation, l'électrode active 5 de l'élément sensible capacitif 3 doit être réalisée de façon à tenir en température, au cours du fonctionnement du moteur et à résister aux agressions de l'huile diesel usée. De plus, au niveau de la connexion de cette électrode active 5 avec le circuit électronique de traitement, une étanchéité de traversée doit être assurée, de même qu'au niveau du câble blindé de jonction entre cette électrode 5 et ce circuit, pour éviter les fuites d'huile.

Le circuit électronique de traitement est destiné à mesurer la concentration de matières charbonneuses dans l'huile à partir de la capacité existant entre les électrodes 4 et 5 de l'élément sensible capacitif 3. Les matières charbonneuses existant dans l'huile en raison de son usure modifient sa constante diélectrique de sorte que la capacité entre les électrodes varie. Le circuit électronique, qui peut être par exemple un transducteur capacité-fréquence tel que celui objet de la demande de brevet français publiée sous le numéro 2 576 421 au nom de la demanderesse, convertit les grandeurs physiques délivrées par l'élément sensible capacitif 3 en variation de fréquences. L'élément sensible capacitif 3 présente une réponse linéaire de 0,5 à 6 % de matières charbonneuses, avec une variation de la constante diélectrique de 10 % pour 1 % de matières charbonneuses.

Grâce à ce dispositif selon l'invention, on peut décider de l'opportunité d'une vidange d'huile soit en fonction d'un pourcentage prédéterminé de matières charbonneuses, soit en fonction d'un kilométrage établi. On peut ainsi décider de la nécessité de faire la vidange lorsque le pourcentage de matières charbonneuses atteint 3 % ou lorsque le kilométrage est supérieur ou égal au kilométrage parcouru affecté d'un terme correctif.

**Revendications**

1/ Dispositif de mesure de la concentration de matières charbonneuses dans une huile pour moteur Diesel, comportant un boîtier renfermant un élément sensible capacitif destiné à être immergé dans l'huile, et dont la capacité varie en fonction de la constante diélectrique de l'huile, caractérisé en ce que cet élément sensible (3) est constitué par deux électrodes parallèles, l'une d'elles connectée à la masse et dite de blindage (4) est réalisée par deux plaques métalliques parallèles reliées entre elles et l'autre dite active (5) placée entre ces deux plaques et isolée électriquement sur presque toute sa surface est reliée à un circuit électronique de traitement (7) destiné à mesurer la concentration de matières charbonneuses dans l'huile à partir de la capacité existante entre lesdites électrodes.

2/ Dispositif selon la revendication 1, caractérisé en ce qu'il est monté en dérivation du circuit d'huile par une entretoise avec piquage, placée entre le carter et le filtre à huile et en ce qu'il comporte un boîtier (1) dont une partie (2) renferme l'élément sensible capacitif (3) susceptible d'être immergé totalement dans l'huile et dont une autre partie (8) étanche renferme le circuit électronique de traitement (7), un premier flexible (9) reliant le carter du moteur au boîtier (1) au niveau d'un gicleur (11) prévu dans sa paroi et un second flexible (10)] évacuant l'huile vers le carter inférieur.

3/ Dispositif selon la revendication 2, caractérisé en ce que l'élément sensible (3) est situé dans la partie (2) du boîtier (1) un peu en dehors de l'écoulement de l'huile entre les deux flexibles (9) et (10).

4/ Dispositif selon la revendication 1, caractérisé en ce qu'il est monté en série avec le circuit d'huile et en ce qu'il comporte un boîtier disposé directement entre le carter et le filtre à huile, l'élément sensible (3) étant placé dans une cavité en couronne dans laquelle circule l'huile venant du carter avant d'y retourner par une cavité cylindrique centrale.

5/ Dispositif selon la revendication 4, caractérisé en ce que les électrodes (4 et 5) de l'élément sensible capacitif (3) sont en forme de cylindres concentriques placés dans une cavité en couronne (22) du boîtier (20).

6/ Dispositif selon la revendication 4, caractérisé en ce que les électrodes (4 et 5) de l'élément sensible capacitif (3) sont en forme de couronnes parallèles et concentriques situées dans une cavité (32) en couronne du boîtier (30).

7/ Dispositif selon la revendication 4 à 6, caractérisé en ce que l'électrode active (5) est connectée électriquement à un circuit électronique de traitement par l'intermédiaire d'un câble blindé (36).

8/ Dispositif selon l'une des revendications 4 à 6, caractérisé en ce que le circuit électronique (37) de traitement est accolé au boîtier (30) contenant l'élément sensible (3) capacitif qui est relié électriquement directement au circuit (37).

9/ Dispositif selon la revendication 1, caractérisé en ce qu'il est monté en série avec le circuit d'huile et en ce qu'il comporte un boîtier disposé directement entre le carter et le filtre à huile, l'élément sensible (3) étant placé dans une cavité en couronne (62) dans laquelle circule l'huile venant du carter après séjour dans une chambre de tranquilisation (65) existant entre une cavité centrale (61) et la paroi du tube de sortie du filtre à huile, l'huile sortant du dispositif par un gicleur (67) prévu dans la paroi extérieure du boîtier dans un flexible (68) qui la conduit au carter inférieur du moteur.

10/ Dispositif selon la revendication 9, caractérisé en ce que l'électrode active (5) est reliée par un câble blindé (69) à un circuit électronique de traitement placé loin des températures élevées du moteur en fonctionnement.

11/ Dispositif selon l'une des revendications précédentes, caractérisé en ce que l'électrode active (5) est en cuivre recouverte sur ses deux faces d'une couche de matériau isolant, tel que du Kapton.

0291363

FIG.2

FIG.1

**FIG.3**

**FIG.4**

**FIG.7**

**FIG.8**

FIG.6

FIG.5

30

45

31

5

36

46

45

31

33

5

35

34

4

32

36

0291363

**FIG.9**

0291363

FIG.10

0291363

0291363

FIG.12

FIG.11

## FIG.13

60

4

62

64

65

61

45

5

46

69

## FIG.14

60

62

66

61

65

## FIG.15

60

62

66

61

0291363